# EUROPEAN PATENT SPECIFICATION

(11) **EP 3 743 034 B1**
(45) Date of publication and mention of the grant of the patent: **09.02.2022**
(21) Application number: 18836416.0
(22) Date of filing: 28.12.2018
(51) Int. Cl.: A61J 1/03, A61J 7/02, A61J 7/04

(54) **SYSTEM FOR MONITORING AND/OR CONTROLLING MEDICATION-TAKING BY A PATIENT AND METHOD ASSOCIATED THEREWITH**
SYSTEM ZUM ÜBERWACHEN UND / ODER KONTROLLIEREN DER MEDIKAMENTENEINNAHME DURCH EINEN PATIENTEN UND ZUGEHÖRIGES VERFAHREN
SYSTÈME DE SURVEILLANCE ET/OU DE CONTRÔLE DE PRISE DE MÉDICAMENT PAR UN PATIENT ET PROCÉDÉ ASSOCIÉ À CELUI-CI

(30) Priority: 25.01.2018 IT 201800001807
(43) Date of publication of application: 02.12.2020
(73) Proprietor: Palladio Group S.p.A., 36031 Dueville (VI) (IT); Omnys S.r.l., 36100 Vicenza (VI) (IT)
(72) Inventor: IANNIZZOTTO, Gabriele, Dublin (IE); MARCHI, Antonio, 36100 Vicenza (VI) (IT); LATINI, Mauro Silvano, 36065 Mussolente (VI) (IT); POZZA, Davide, 36056 Tezze sul Brenta (VI) (IT)
(74) Representative: Ziliotto, Tiziano
(86) International application number: PCT/IB2018/060676
(87) International publication number: WO 2019/145776

(56) References cited:
- EP-A1- 3 061 438
- WO-A1-2012/136989
- WO-A1-2017/130207
- US-A1- 2017 004 284
- US-A1- 2017 248 401

## Description

The present invention concerns a system designed to monitor and/or control medication-taking by a patient.

More specifically, the present invention concerns a system designed to monitor and/or control the taking of medications in the form of pills by a patient.

Furthermore there is disclosed also a method for managing said system for monitoring and/or controlling correct medication-taking by a patient.

### DESCRIPTION OF THE STATE OF THE ART

Devices are known, which serve as an aid for the taking of medications by patients suffering from a disease.

Document US4,660,991 describes a device which is suited to be associated with a pack of pills, commonly known as blister pack. The device is suited to receive said blister pack and is capable of periodically warning the patient when he/she must take the medication, for example through an audio or video signal. The device is capable of detecting when the patient extracts the pill from the blister pack by means of suitable sensors and is capable of storing the time when the pills are taken, of displaying these values and, if necessary, of signalling that the pill has not been taken at the scheduled time.

Further pills detection systems are known from documents WO 2017/130207 A1, EP 3 061 438 A1, US 2017/004284 A1, US 2017/247401 A1 and WO 2012/136989 A1.

It is the object of the present invention to improve the systems of the known type in order to allow the correct taking of medications by patients and the control of said medication-taking.

It is another object of the present invention to propose a solution which allows third-party operators in addition to the patient, for example the doctor who prescribed said medications or a relative who takes care of the patient, to monitor and, if necessary, adjust the correct taking of medications by patients.

### SUMMARY OF THE PRESENT INVENTION

The present invention is based on the general consideration that it is desirable to provide a system for monitoring and/or controlling the taking of one or more medications packaged in a blister pack by a patient, said system comprising an identification unit that serves to identify the condition of the blister pack and the wireless transmission of the information regarding the condition of the blister pack to a service provider.

The invention is defined by the appended claims. Hereinafter several embodiments not falling under the subject-matter defined by the claims are disclosed.

According to a first aspect the subject is a system for monitoring and/or controlling the taking of one or more medications by a patient, wherein said one or more medications are packaged in a blister pack, said system comprising:
- an identification unit suited to identify the condition of said blister pack, said identification unit being suited to identify how many and/or which of said one or more medications are extracted from said blister pack;
- a management unit communicating with said identification unit and suited to receive and process information transmitted by said identification unit and regarding how many and/or what medications have been extracted from said blister pack;
- a wireless communication unit communicating with said management unit and suited to transmit and/or receive wireless data;
- a service provider suited to receive said wireless data from said wireless communication unit and/or transmit wireless data to said wireless communication unit.

In a preferred embodiment, the system comprises also a casing suited to receive said blister pack, said casing comprising detection means suited to detect how many and/or which of said one or more medications are extracted from said blister pack.

Preferably, the system comprises a connection module interposed between the casing and the identification unit.

According to a preferred embodiment the identification unit is part of said casing and communicates with the management unit wirelessly.

In a preferred embodiment, the connection module, the identification unit, the management unit and the wireless communication unit belong to a device which is suited to be used by the patient and to be connected to the casing that receives the blister pack.

Preferably, the device comprises an interface for the patient. More preferably, the interface comprises a display unit for the patient.

According to a preferred embodiment of the invention, the management unit and the wireless communication unit belong to a device, preferably a smartphone, more preferably a smartphone with a dedicated APP.

Preferably, the device communicates wirelessly with the identification unit that identifies the condition of the blister pack, preferably through a type of communication belonging to the group of types of communication including the following: BT communication, NFC communication, ZIGBEE communication, WIFI communication, UHF communication.

In a preferred embodiment of the invention, the identification unit is integrated in said blister pack.

Preferably, the service provider manages one or more interfaces for third-party operators.

The wireless communication unit and the service provider preferably communicate through a wide range communication network, for example one among the following networks: GSM, 3G, 4G, 5G, Sigfox, LoRa.

In a preferred embodiment, the management unit comprises storage means suited to store the patient's details.

According to a preferred embodiment the blister pack comprises an identification system suited to generate an identification code for the type of medication received inside it.

According to a preferred embodiment the casing comprises an identification system suited to generate an identification code suited to identify the type of blister pack or the type of medication that it can receive.

In a preferred embodiment, said identification system comprises a bar code or a RFID or a NFC.

Preferably, the identification unit comprises means for reading said identification code. More preferably, the reading means comprise a bar code reader, a RFID reader, a NFC reader.

According to a preferred embodiment the management unit and/or the service provider is suited to determine when said medications have been extracted from the blister pack.

Preferably, said blister pack comprises one or more deformable cavities closed by one or more respective covers.

According to a second aspect the subject of the same is a method for monitoring and/or controlling the taking of one or more medications by a patient within a system of the type described above, comprising the following steps:
- by means of said identification unit, identifying how many and/or which of said one or more medications are extracted from said blister pack;
- by means of said management unit, processing the information transmitted by said identification unit regarding how many and/or what medications have been extracted from said blister pack;
- by means of said wireless communication unit, transmitting the information regarding how many and/or what medications have been extracted from said blister pack to said service provider.

In a preferred embodiment, the method comprises a step during which information is communicated to the patient based on the result produced by the information processed by the management unit.

In a preferred embodiment, the method comprises a step during which data are transmitted to the management unit by the service provider through said wireless communication unit.

According to a preferred embodiment the data transmitted by said service provider modify the information concerning the patient that are contained in said management unit.

In a preferred embodiment, the service provider interacts with one or more third-party operators.

According to a preferred embodiment the method comprises a step during which the management unit and/or the service provider determine/s when said medications have been extracted from the blister pack.

Preferably, the method comprises a step during which the type of blister pack is identified univocally.

According to a third aspect the subject of the same is a casing suited to receive a blister pack for one or more medications, said casing comprising detection means suited to detect when one of said one or more medications is extracted from said blister pack, wherein the casing comprises an identification unit that identifies the condition of said blister pack, said identification unit being suited to identify how many and/or which of said one or more medications are extracted from said blister pack and wherein the casing communicates with another unit wirelessly.

According to a fourth aspect the subject of the same is a blister pack for one or more medications, comprising:
- detection means suited to detect when one of said one or more medications is extracted from said blister pack;
- an identification unit that identifies the condition of said blister pack, said identification unit being suited to identify how many and/or which of said one or more medications are extracted from said blister pack, said identification unit being suited to communicate with another unit wirelessly.

Preferably, said blister pack comprises one or more deformable cavities closed by one or more respective covers.

In a preferred embodiment, the blister pack is suited to determine when said medications have been extracted from the blister pack itself.

### BRIEF DESCRIPTION OF THE DRAWINGS

Further advantages, objects and characteristics, as well as further embodiments of the present invention, are defined in the claims and will be illustrated in the following description, with reference to the enclosed drawings; in the drawings, corresponding or equivalent characteristics and/or components of the present invention are identified by the same reference numbers. More specifically, in the drawings:
- Figure 1 shows a schematic view of a system according to the present invention;
- Figure 2 shows a system according to a preferred embodiment of the present invention;
- Figure 3 shows an axonometric view of two elements of the system shown in Figure 2 separate from each other and ready to be coupled with each other;
- Figure 4 shows an axonometric view of the elements of Figure 3 coupled together;
- Figure 5 shows an axonometric view of the elements of Figure 4 associated with a device carried out according to a preferred embodiment of the present invention;
- Figure 6 shows the elements of Figure 5 in a particular condition of use;
- Figure 7 shows a variant embodiment of the system of Figure 2;
- Figure 8 shows another variant embodiment of the system of Figure 2;
- Figure 9 shows a further variant embodiment of the system of Figure 2.

### DETAILED DESCRIPTION OF PREFERRED EMBODIMENTS OF THE PRESENT INVENTION

The present invention will be further clarified by means of the following detailed description of its preferred embodiments illustrated in the drawings. It should however be noted that the present invention is not limited to the particular embodiments described below and illustrated in the drawings; rather, it should be noted that the embodiments described below and illustrated in the drawings clarify some aspects of the present invention, the scope of which is defined in the claims. The present invention must therefore be considered as including all those variants and/or modifications that will be clear for the expert in the art.

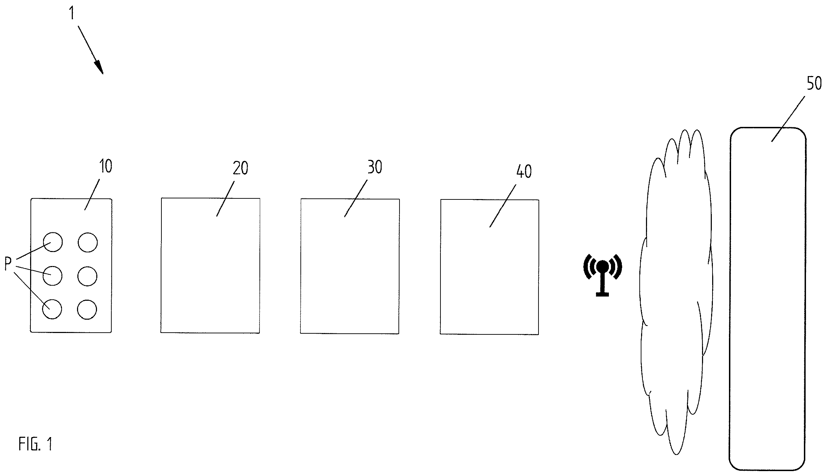

Figure 1 shows a general diagram of the monitoring and/or control system 1 that is the subject of the present invention.

The system 1 according to the present invention concerns the taking of one or more medications P by a patient, preferably medications in the form of pills P, said medications P being packaged in a blister pack 10.

As is known, correct medication-taking is a delicate and important aspect, for example in the treatment or prevention of diseases. In many cases, the patient is required to follow a precise schedule in the taking of the medication, in terms of both quantity and times.

In general terms, therefore, a patient's profile can be defined as the combination of information which includes the patient's identifier, for example name and surname or taxpayer identification number, the type of medication to be taken and the times when said medication must be taken.

The present description makes reference to medications packaged in blister packs.

As is known, a blister pack comprises one or more deformable cavities closed by one or more respective covers, the medication being received in said cavities. The cavity preferably comprises a sufficiently deformable material, such as plastic and aluminium, and the cover is preferably made of yielding paper, plastic or aluminium.

According to the above, in the context of the present invention a medication is generally intended to be a dose of a drug suited to be received in a blister pack, such as a pill, a dose of a drug available in the form of granules or of a powder, etc.

The medication P is extracted from the blister pack 10 by pressing against the deformable cavity with a finger: the pressure exerted by the finger on the medication P breaks the cover and allows the medication to be expelled.

With reference to Figure 1, the monitoring system 1 according to the invention preferably comprises an identification unit 20 suited to identify the condition of the blister pack 10, a management unit 30, a wireless communication unit 40 and a service provider 50.

The identification unit 20 that identifies the condition of the blister pack 10 preferably represents a unit capable of determining how many and/or which medications P are extracted from the blister pack 10.

The management unit 30, which communicates with the identification unit 20, receives and processes the information transmitted by the identification unit 20 regarding how many and/or which medications P have been extracted from the blister pack 10.

In a preferred embodiment, the management unit 30 is also capable of determining when the medications P were extracted from the blister pack 10. The wireless communication unit 40, which communicates with the management unit 30, transmits and/or receives wireless data to/from the service provider 50. More specifically, it transmits data concerning how many and/or which medications P were extracted from the blister pack 10.

In a preferred embodiment, the management unit 30 transmits also data concerning when the medications P were extracted from the blister pack 10.

The service provider 50 receives said wireless data from the wireless communication unit 40 and/or transmits wireless data to the wireless communication unit 40. More specifically, it receives data concerning how many and/or which medications P were extracted from the blister pack 10.

In a preferred embodiment, the service provider 50 receives also data concerning when the medications P were extracted from the blister pack 10.

In a further preferred embodiment, the service provider 50 itself determines when the medications P were extracted from the blister pack 10.

The wireless communication unit 40 and the service provider 50 preferably communicate through a wide range communication network, preferably a network of the type GSM, 3G, 4G, 5G, Sigfox, LoRa.

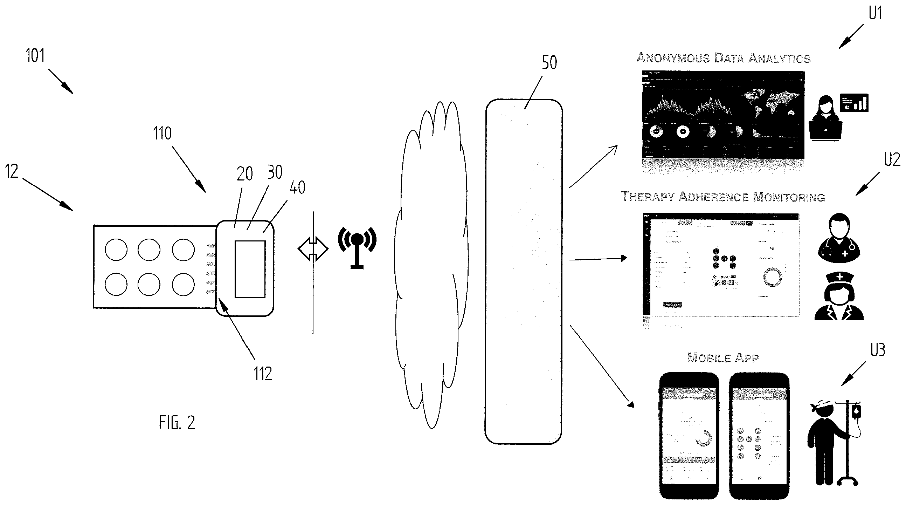

As explained in greater detail below, the service provider 50, which has said data available, is preferably capable of managing one or more interfaces for third-party operators, for example the operators indicated by U1, U2, U3 in Figures 2, 7, 8 and 9.

A third-party operator may be, for example, the pharmaceutical company U1, which produces a specific medication P and may be interested in knowing the quantity of said medication P that has been taken or the period when it was taken, and/or a doctor U2, who prescribed and/or follows the patient's therapy, or any other person U3 involved, for example a relative or the patient himself/herself. Furthermore, one of said third-party operators U1, U2, U3 can interact with the management unit 30 through the service provider 50, for example for the purpose of controlling and/or setting the functions that are specific of the management unit 30.

Figure 2 shows a first possible embodiment of the system 101 which is the subject of the invention.

In the drawing, component parts corresponding or equivalent to those indicated in the general diagram of Figure 1 are identified by the same reference numbers.

The system 101 comprises a set 12 defined by a casing 14 which receives the blister pack 10, a device 110 suited to be used by the patient and suited to receive the set 12, a service provider 50 and three interfaces for third-party operators U1, U2, U3.

The device 110 preferably incorporates the identification unit 20 that identifies the condition of the blister pack 10, the management unit 30 and the wireless communication unit 40 previously described.

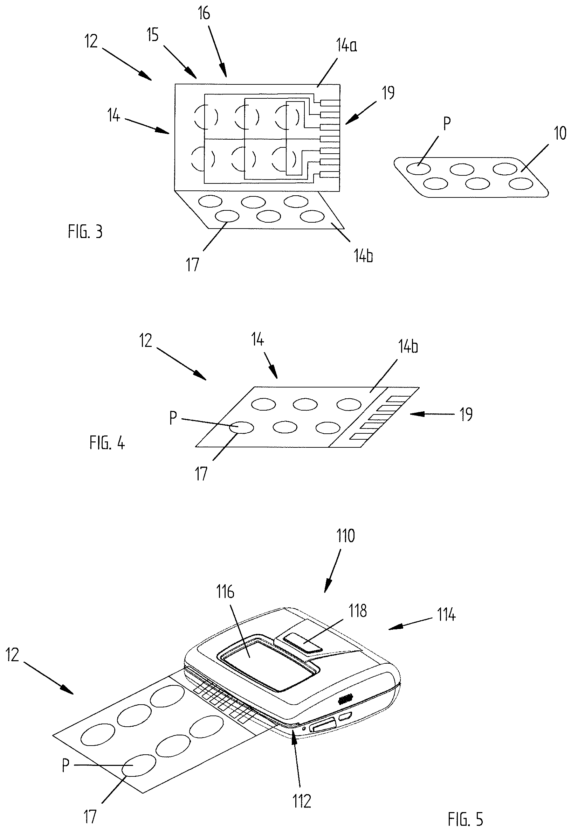

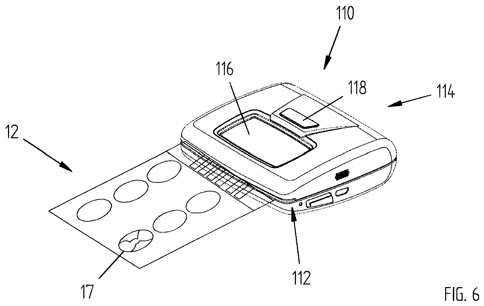

The device 110, as shown in particular in Figures 5 and 6, is suited to receive the set 12 comprising the casing 14 with the blister pack 10 inside it.

The casing 14, as shown in Figure 3, preferably comprises detection means 15 suited to detect how many and/or which of said one or more medications P are extracted from the blister pack 10.

According to the invention, the casing 14 comprises a first substrate 14a on which a layout, schematically illustrated and indicated by 16 in Figure 3, is printed with an ink of the conductive type, and successively die-cut in the format suited to receive the blister pack 10.

In a preferred embodiment, the layout 16 is printed on both faces of the first substrate 14a.

The casing 14 comprises a second substrate 14b on which holes 17 are defined, said holes being preferably made in a position which is aligned with the position of the medications P in the blister pack 10 when the latter is received in the casing 14.

At the moment of extraction of a medication from the blister pack 10, the patient presses with a finger against the deformable cavity of the blister pack 10 and the pressure exerted by the finger on the medication P causes the breakage of the cover of the blister pack 10 itself and at the same time the breakage of one or more tracks of said layout 16 associated with the casing 14. This leads to a modification of the layout 16, which makes it possible to detect which medication P contained in the blister pack 10 has been extracted.

Preferably, the substrates 14a, 14b are made of paper, or cardboard, plastic, plastic film, aluminium, metallic foils, coupled materials, etc. The shapes and dimensions of the substrates can be different and suited to allow the blister pack 10 to be correctly received therein. The conductive ink can have different origins and colours, with a conductive base constituted by metal powder or alloys such as gold, silver, copper alloys and so on, or by other conductive elements dispersed in vehicles suitable for application with traditional or digital printing systems.

The printing techniques used for the application of the ink and the composition of the layout are preferably the following, direct or indirect: embossing, lithography, screen printing, intaglio printing such as, for example, letterpress printing, flexography, offset printing, serigraphy, rotogravure; of the hot or cold transfer type; of the digital type.

The ink can be preferably protected with paints, inks, decorative prints, lamination films, or coupled with materials such as paper, cardboard, plastic film, metallic foils.

Furthermore, preferably, the casing 14 can be directly coupled with the blister pack 10 or can be available in the existing package so that it can be coupled by the patient.

The casing 14 is preferably coupled with the blister pack 10 by means of glues, double-sided adhesives, adhesive labels.

In a preferred embodiment, the set 12 may comprise destruction systems suited to avoid any re-use of the casing 14 with other blister packs 10, for example a system that causes the irreversible breakage of the layout 16 so as to make it unusable if the blister pack 10 is extracted from the casing 14.

In order to allow the coupling between the casing 14 and the device 110 to be carried out, terminals 19 are preferably defined which are conveniently connected to the detection layout 16.

The device 110 in turn comprises a connection module 112 providing an interface with said terminals 19. The connection module 112 communicates with the identification unit 20.

The connection module 112 provides an interface with the terminals 19 of the casing 14 and is capable of reading the information present on the detection layout 16. In a preferred embodiment, the connection module 112 is capable of reading the tracks both on the upper side and on the underside in the case where both faces of the first substrate 16a are provided with a layout.

In a preferred embodiment, the connection module 112 comprises a connector with bayonet joint closing like an alligator's mouth.

The connection module 112 is thus capable of reading the condition of the blister pack 10 by means of the casing 14, meaning that it is capable of reading how many and/or which medications P were extracted from the blister pack 10.

In a preferred embodiment, the casing 14 comprises a unique identification code capable of identifying the type of blister pack 10 or the type of medication that it can receive.

The identification code preferably comprises a specific code printed on the layout.

In variant embodiments, the identification code can be of a different type, for example a bar code, an RFID code, an NFC code, etc.

In a different preferred embodiment, the blister pack 10 itself comprises a unique identification code which is suited to identify the type of medication contained therein.

The identification code preferably comprises a specific code such as, for example, a bar code, an RFID code, an NFC code, etc.

The device 110 will be preferably provided with reading means capable of detecting said identification code associated with the casing 14 or with the blister pack 10.

The reading means preferably comprise a bar code reader, an RFID reader, an NFC reader, etc.

The device 110 furthermore comprises an interface 114 for the patient. The interface 114 preferably comprises a display unit 116, or display, and a control button 118. In different embodiments, the interface 114 may comprise an acoustic alarm and/or a voice warning device.

According to the above, the management unit 30 of the device 110, which communicates with the identification unit 20, receives and processes the information transmitted by the identification unit 20 regarding how many and/or which medications P were extracted from the blister pack 10.

For example, as illustrated in Figure 6, the device 110 is capable of detecting that a medication P was extracted from the blister pack 10.

Furthermore, the device 110, and in particular the management unit 30, as explained above, is capable of determining also when the medications P were extracted from the blister pack 10, for example thanks to an internal clock. Preferably, the management unit 30 comprises storage means suited to store one or more patient profiles.

The patient profile is capable of identifying the patient, for example through his/her name and surname or tax code, the type of medication to be taken and preferably the medicine taking times.

The management unit 30 provides for coordinating all the aspects of the operation of the device 110. The management unit 30 preferably provides for:
- managing the user interface 114 (for example, the display 116);
- managing the taking of the medication/medications by the patient and managing the alarms or warnings for the patient, preferably through the display 116 and/or acoustic signals and/or voice instructions;
- managing the correct use and/or operation of the device 110, for example controlling the temperature, dealing with any operation anomalies, etc.;
- checking the correct or failed taking of the medication, with consequent notification to the service provider 50 through the wireless communication unit 40;
- managing communication with the wireless communication unit 40;
- managing the casings 14 and/or blister packs 10 that the patient can use through the device 110;
- sending an SOS alarm to the service provider 50 and/or to one of the third-party operators involved, who may intervene (preferably through a specific emergency button present on the device 110).

Furthermore, the management unit 30 is capable of receiving and managing the information transmitted by the service provider 50, such as, for example, the configuration parameters of the device 110, the updates of the user profile/profiles, etc. Such updates can be preferably requested by one or more of the third-party operators U1, U2, U3, for example in the case where the doctor U2 who follows the patient U2 deems it necessary to modify the administration times or the type of medication.

Such updates can also be preferably requested by the service provider 50 itself. A possible operating mode of the system 1 according to the invention includes the following steps.

First of all, there is an initialization procedure for the device 110 through the registration of the patient, which is intended to associate a specific device 110 with a specific patient, for example by entering his/her name and surname or tax code. This data is advantageously sent to the service provider 50 and/or to one or more of the third-party operators U1, U2, U3, which therefore will be aware that a given device is associated with a specific patient.

At this point a patient profile is created, in which the type of medication to be taken by the patient is identified. This profile can be directly stored in the device 110 through the specific interface 114, for example by the patient himself/herself, or remotely by the service provider 50 and/or by one of the third-party operators U1, U2, U3. For example, the doctor U2 can define the profile of a patient by specifically indicating that the patient needs to take an antibiotic pill every 4 hours.

The patient then introduces the blister pack 10 containing the prescribed medication (for example, the antibiotic) inside the casing 14. Then, he/she proceeds to take the medication, extracting it from the blister pack 10 and thus from the set 14.

He/she successively introduces the set 14 inside the device 110, as shown for example in Figure 6, and the device 110 thus detects that a medication P was extracted and presumably ingested by the patient.

The device 110 is able to immediately provide useful information to the patient, such as:
- notification stating "blister pack connected and authorized";
- notification stating "blister damaged";
- notification stating "medication taken";
- expected time when the next dose of the medication should be taken;
- notification stating "blister pack extracted and containing no more pills";
- notification stating "blister pack extracted and still containing pills";
- warning that new pills must be bought;
- information indicating where the new pills can be bought, and possibly the nearest pharmacies.

Furthermore, the device 110 can:
- allow the user to buy the medication directly from the device itself;
- propose surveys and questionnaires related to the efficacy of the medication and the progress of the treatment;
- make it possible to signal any side and/or undesired effects;
- confirm the transmission of an SOS alarm.

Other information can be displayed, for example a warning indicating that the battery is down or that there are connectivity problems towards the service provider.

Furthermore, to advantage, the device 110 transmits the information that the medication has been taken to the service provider 50 and/or to one of the third-party operators U1, U2, U3. In particular, the doctor U2 can monitor the correct progress of the treatment and, if necessary, decide to modify it, for example by changing the administration interval between two pills. The doctor U2 can remotely update the patient profile directly inside the device 110.

Other advantageous aspects related to the operation of the system 1 include the possibility to directly check whether the set being used, that is, the casing 14 and/or the blister pack 10, is exactly the one requested by the treatment.

Thanks to the presence of the identification codes associated with the casing and/or the blister pack, as described above, the device 110 is capable of double-checking whether the medication being taken corresponds to that prescribed for the treatment according to the patient profile stored inside it.

This may lead to immediate notification to the patient through the interface 114 and/or notification to the service provider 50 and/or to one of the third-party operators U1, U2, U3. For example, advantageously, the doctor and/or a relative can intervene immediately in order to remedy an anomalous situation.

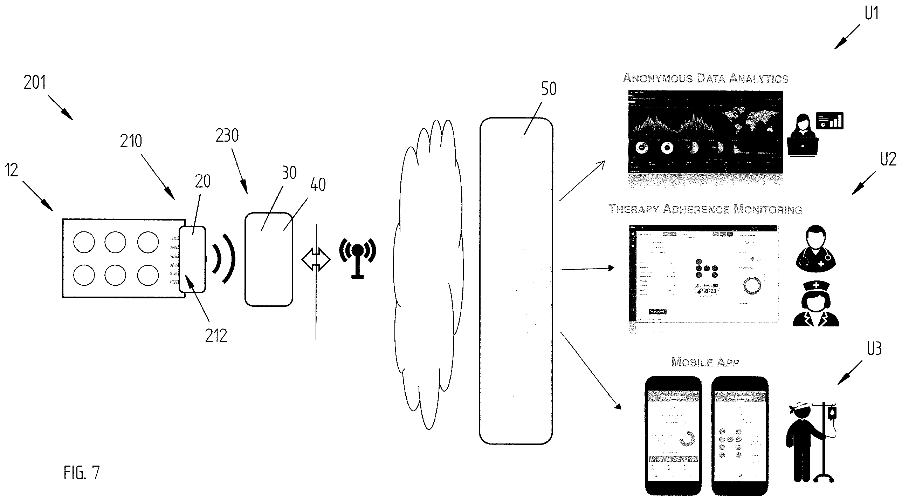

Figure 7 shows another possible embodiment of the system 201 which is the subject of the invention.

In the drawing, component parts corresponding or equivalent to those indicated in the general diagram of Figure 1 and/or related to the system previously described with reference to Figure 2 are identified by the same reference numbers.

This embodiment of the system 201 differs from the system previously described in that all the modules 20, 30 and 40, 112 previously incorporated in the device 110 are now integrated in two separate devices 210 and 230 which communicate with each other. From the functional point of view, the two devices 210 and 230 as a whole serve the same functions as the device 110, in accordance with the description provided above.

The system 201 thus preferably comprises a set 12 defined by a casing 14 which receives the blister pack 10, a first device 210 suited to receive the set 12, a second device 230, a service provider 50 and three interfaces for third-party operators U1, U2, U3.

The first device 210 preferably incorporates the identification unit 20 that identifies the condition of the blister pack 10 and the connection module 212 for interfacing with the set 12.

The second device 230 preferably incorporates the management unit 30 and the wireless communication unit 40 previously described.

The first and the second device 210, 230 preferably communicate with each other through a wireless connection. The two devices 210, 230 preferably communicate through a technology of the known type, for example a BT, NFC, ZIGBEE, WIFI, UHF technology, etc.

In a preferred embodiment, the second device 230 comprises a cellular phone, preferably a smart phone, provided with a specific program installed therein, for example an App suited to serve the same functions as the management unit 30 and the wireless communication unit 40.

The display of the cellular device preferably serves as an interface towards the user.

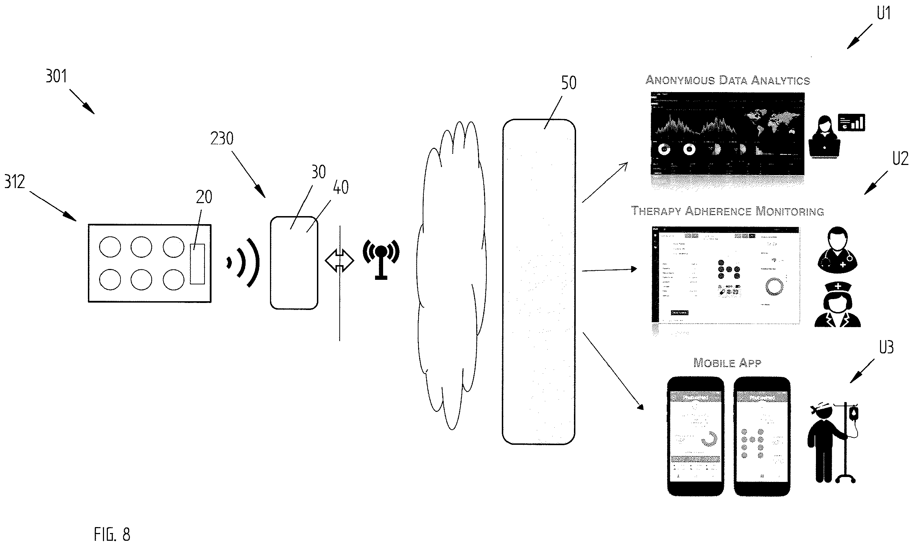

Figure 8 shows another possible embodiment of the system 301 which is the subject of the invention.

In the drawing, component parts corresponding or equivalent to those indicated in the general diagram of Figure 1 and/or related to the systems 101 and 201 previously described with reference to Figures 2 and 7 are identified by the same reference numbers.

Such embodiment of the system 301 differs from the systems previously described in that the identification unit 20 that identifies the condition of the blister pack 10, the management unit 30 and the wireless communication unit 40 are distributed differently.

The system 301 preferably comprises a set 312 defined by a casing 14 which receives the blister packs 10 and in which the identification unit 20 is integrated. In this case the connection module is omitted, since the identification unit 20 is integrated in the casing 14.

The system 301 preferably comprises also a second device 230, a service provider 50 and three interfaces for third-party operators U1, U2, U3.

The second device 230 is a device carried out as described above with reference to the embodiment of Figure 7 and preferably incorporates the management unit 30 and the wireless communication unit 40 previously described.

The identification unit 20 of the set 312 and the second device 230 preferably communicate with each other through a wireless connection. Preferably, communication takes place through a technology of the known type, for example a BT, NFC, ZIGBEE, WIFI, UHF technology, etc.

In a preferred embodiment, as already explained above, the second device 230 comprises a cellular phone, preferably a smart phone, provided with a specific program installed therein, for example an App suited to serve the same functions as the management unit 30 and the wireless communication unit 40.

The display of the cellular device preferably serves as an interface towards the user.

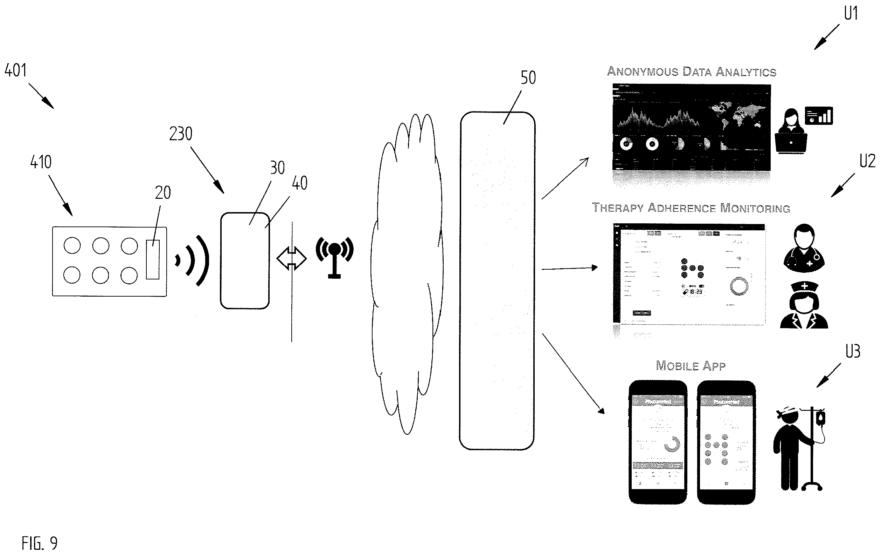

Figure 9 shows another possible embodiment of the system 401 which is the subject of the invention.

In the drawing, component parts corresponding or equivalent to those indicated in the general diagram of Figure 1 and/or related to the systems 101, 201 and 301 previously described with reference to Figures 2, 7 and 8 are identified by the same reference numbers.

Such embodiment of the system 401 differs from the previous embodiment described with reference to Figure 8 especially in that the identification unit 20 is integrated directly in the blister pack 410. In this embodiment, the casing suited to receive the blister pack is not present.

The system 401 thus preferably comprises a blister pack 410 that integrates the identification unit 20, a second device 230, a service provider 50 and three interfaces for third-party operators U1, U2, U3.

The second device 230 is a device carried out as described above with reference to the embodiments of Figures 7 and 8 and preferably incorporates the management unit 30 and the wireless communication unit 40 previously described.

The identification unit 20 that identifies the blister pack 410 and the second device 230 preferably communicate with each other through a wireless connection. Preferably, communication takes place through a technology of the known type, for example a BT, NFC, ZIGBEE, WIFI, UHF technology, etc.

In a preferred embodiment, as already explained above, the second device 230 comprises a cellular phone, preferably a smart phone, provided with a specific program installed therein, for example an App suited to serve the same functions as the management unit 30 and the wireless communication unit 40.

The display of the cellular device preferably serves as an interface towards the user.

As far as the blister pack 410 is concerned, it will be provided with suitable electronics installed thereon and suited to detect any breakage of the covers of the cavities when the medication is extracted. A suitable energy source will be provided in order to supply power to said electronics, such as, for example, batteries or photovoltaic cells.

In a preferred embodiment, the blister pack 410 comprises a unique identification code suited to identify the type of medication contained in its cavities.

The identification code preferably comprises a specific code such as, for example, a bar code, an RFID code, an NFC code, etc.

The operating modes of the systems according to Figures 7, 8 and 9 will substantially be the same as those described above with reference to the system shown in Figure 1.

The extraction of a medication from the blister pack and the assumed taking of the same are detected by the system, which is capable of providing information to the patient himself/herself or to a third party involved. Furthermore, the system makes it possible to intervene even remotely in order to check the correct progress of the treatment and to make any adjustments.

It has thus been shown, by means of the description provided, that the system carried out according to the present invention allows the set objects to be achieved. More specifically, the monitoring and/or control system according to the present invention makes it possible to monitor and, if necessary, adjust the taking of medications by patients.

Even if the present invention has been illustrated above by means of the detailed description of some embodiments represented in the drawings, the present invention is not limited to the embodiments described above and represented in the drawings; on the contrary, further variants of the embodiments described above fall within the scope of the present invention, which is defined in the claims.

## Claims

1. System (1; 101; 201; 301; 401) for monitoring and/or controlling the taking of one or more medications (P) by a patient, said one or more medications (P) being contained in a blister pack (10; 410), said system (1; 101; 201; 301; 401) comprising:
- an identification unit (20) suited to identify the condition of said blister pack (10; 410), said identification unit (20) being suited to identify how many and/or which of said one or more medications (P) are extracted from said blister pack (10; 410);
- a management unit (30) communicating with said identification unit (20) and suited to receive and process information transmitted by said identification unit (20) and regarding how many and/or what medications (P) have been extracted from said blister pack (10; 410);
- a wireless communication unit (40) communicating with said management unit (30) and suited to transmit and/or receive wireless data;
- a service provider (50) suited to receive said wireless data from said wireless communication unit (40) and/or transmit wireless data to said wireless communication unit (40);
a casing (14) suited to receive said blister pack (10), said casing (14) comprising detection means (15) suited to detect how many and/or which of said one or more medications (P) are extracted from said blister pack (10), **characterized by**
said casing (14) further comprising a first substrate (14a) on which a layout (16) is printed with a conductive type ink and which is die-cut in a format suited to receive said blister pack (10),
said casing (14) further comprising a second substrate (14b) on which holes (17) are defined, said holes being made in a position which is aligned with a position of the one or more medications (P) in the blister pack (10) when the blister pack (10) is received in the casing (14).

2. System (1; 101; 201) according to claim 1, **characterized in that** it comprises a connection module (112; 212) interposed between said casing (14) and said identification unit (20).

3. System (301) according to claim 1 or 2, **characterized in that** said identification unit (20) is part of said casing and communicates wirelessly with said management unit (30).

4. System (1; 101) according to any of the claims from 1 to 2, **characterized in that** said connection module (112), said identification unit (20), said management unit (30) and said wireless communication unit (40) belong to a first device (110) which is suited to be used by said patient and to be connected to said casing (14) receiving said blister pack (10).

5. System (1; 101) according to claim 4, **characterized in that** said first device (110) comprises an interface (114) for said patient.

6. System (1; 101) according to claim 5, **characterized in that** said interface (114) comprises a display unit (116) for said patient.

7. System (201; 301; 401) according to any of the preceding claims, **characterized in that** said management unit (30) and said wireless communication unit (40) belong to a second device (230), preferably a smartphone, more preferably a smartphone with a dedicated APP.

8. System (201; 301; 401) according to claim 7, **characterized in that** said second device (230) communicates wirelessly with said identification unit (20) that identifies the condition of said blister pack (10; 410), preferably through a type of communication belonging to the group of types of communication including the following: BT communication, NFC communication, ZIGBEE communication, WIFI communication, UHF communication.

9. System (401) according to any of the preceding claims, **characterized in that** said identification unit (20) is integrated in said blister pack (10; 410).

10. System (1; 101; 201; 301; 401) according to any of the preceding claims, **characterized in that** said service provider (50) manages one or more interfaces for third-party operators (Ul, U2, U3).

11. System (1; 101; 201; 301; 401) according to any of the preceding claims, **characterized in that** said wireless communication unit (40) and said service provider (50) communicate with each other through a wide range communication network, preferably one among the GSM, 3G, 4G, 5G, Sigfox, LoRa networks.

12. System (1; 101; 201; 301; 401) according to any of the preceding claims, **characterized in that** said management unit (30) comprises storage means suited to store data concerning said patient.

13. System (1; 101; 201; 301; 401) according to any of the preceding claims, **characterized in that** said blister pack (10; 410) comprises an identification system suited to generate an identification code for the type of medication received therein.

14. System (1; 101; 201; 301; 401) according to any of the preceding claims, **characterized in that** said casing (14) comprises an identification system suited to generate an identification code in order to identify the type of blister pack (10; 410) or the type of medication it can receive.

15. System (1; 101; 201; 301; 401) according to claim 13 or 14, **characterized in that** said identification system comprises a bar code or a RFID code or a NFC code.

16. System (1; 101; 201; 301; 401) according to any of the claims from 13 to 15, **characterized in that** said identification unit (20) comprises reading means suited to read said identification code.

17. System (1; 101; 201; 301; 401) according to claim 15, **characterized in that** said reading means comprise a bar code reader, a RFID reader, a NFC reader.

18. System (1; 101; 201; 301; 401) according to any of the preceding claims, **characterized in that** said management unit (30) and/or said service provider (50) is suited to determine when said medications (P) have been extracted from said blister pack (10; 410).

19. Casing suited to receive a blister pack (10) for one or more medications (P), said casing (14) comprising detection means (15) suited to detect when one of said one or more medications (P) is extracted from said blister pack (10),
**wherein** said casing (14) comprises an identification unit (20) that identifies the condition of said blister pack (10), said identification unit (20) being suited to identify how many and/or which of said one or more medications (P) are extracted from said blister pack (10) and
**wherein** said casing (14) communicates wirelessly with an external unit (230) **characterized by**
said casing (14) further comprising a first substrate (14a) on which a layout (16) is printed with a conductive type ink and which is die-cut in a format suited to receive said blister pack (10),
said casing (14) further comprising a second substrate (14b) on which holes (17) are defined, said holes being made in a position which is aligned with a position of the one or more medications (P) in the blister pack (10) when the blister pack (10) is received in the casing (14).

## Patentansprüche

1. System (1; 101; 201; 301; 401) zur Überwachung und/oder Steuerung der Einnahme eines oder mehrerer Medikamente (P) durch eine Patientin/einen Patienten, wobei das besagte eine oder die besagten mehreren Medikamente (P) in einer Blisterpackung (10; 410) enthalten ist/sind, wobei das besagte System (1; 101; 201; 301; 401) folgende Komponenten umfasst:
- eine Identifikationseinheit (20), die geeignet ist, den Zustand der besagten Blisterpackung (10; 410) zu identifizieren, wobei die besagte Identifikationseinheit (20) geeignet ist, zu identifizieren, wie viele und/oder welche der besagten einen oder mehreren Medikamente (P) aus der Blisterpackung (10; 410) entnommen werden;
- eine Managementeinheit (30), die mit der besagten Identifizierungseinheit (20) kommuniziert und geeignet ist, von der besagten Identifizierungseinheit (20) übertragene Informationen zu empfangen und zu verarbeiten, die sich darauf beziehen, wie viele und/oder welche Medikamente (P) aus der besagten Blisterpackung (10; 410) entnommen worden sind;
- eine drahtlose Kommunikationseinheit (40), die mit der besagten Managementeinheit (30) kommuniziert und geeignet ist, drahtlose Daten zu senden und/oder zu empfangen;
- einen Dienstanbieter (50), der geeignet ist, die besagten drahtlosen Daten von der besagten drahtlosen Kommunikationseinheit (40) zu empfangen und/oder drahtlose Daten an die besagte drahtlose Kommunikationseinheit (40) zu senden;
ein Gehäuse (14), das geeignet ist, die besagte Blisterpackung (10) aufzunehmen, wobei das besagte Gehäuse (14) Erfassungsmittel (15) umfasst, die geeignet sind, zu erfassen, wie viele und/oder welche der besagten einen oder mehreren Medikamente (P) aus der Blisterpackung (10) entnommen werden,
**gekennzeichnet dadurch, dass**
das besagte Gehäuse (14) ferner ein erstes Substrat (14a) umfasst, auf das ein Layout (16) mit einer leitfähigen Tinte gedruckt ist und das in einem Format gestanzt ist, das zur Aufnahme der besagten Blisterverpackung (10) geeignet ist, das besagte Gehäuse (14) ferner ein zweites Substrat (14b) umfasst, auf dem Löcher (17) definiert sind, wobei die besagten Löcher in einer Position ausgeführt sind, die mit einer Position des einen oder der mehreren Medikamente (P) in der Blisterpackung (10) ausgerichtet ist, wenn die Blisterpackung (10) in dem Gehäuse (14) aufgenommen wird.

2. System (1; 101; 201) nach Patentanspruch 1, **dadurch gekennzeichnet, dass** es ein Verbindungsmodul (112; 212) umfasst, das zwischen dem besagten Gehäuse (14) und der besagten Identifikationseinheit (20) angeordnet ist.

3. System (301) nach Patentanspruch 1 oder 2, **dadurch gekennzeichnet, dass** die besagte Identifikationseinheit (20) Teil des besagten Gehäuses ist und drahtlos mit der besagten Managementeinheit (30) kommuniziert.

4. System (1; 101) nach jeglichem der Patentansprüche 1 und 2, **dadurch gekennzeichnet, dass** das besagte Verbindungsmodul (112), die besagte Identifikationseinheit (20), die besagte Managementeinheit (30) und die besagte drahtlose Kommunikationseinheit (40) zu einer ersten Vorrichtung (110) gehören, die geeignet ist, von der besagten Patientin/dem besagten Patienten benutzt zu werden und mit dem besagten Gehäuse (14) verbunden zu werden, das die besagte Blisterpackung (10) aufnimmt.

5. System (1; 101) nach Patentanspruch 4, **dadurch gekennzeichnet, dass** die besagte erste Vorrichtung (110) eine Schnittstelle (114) für die besagte Patientin/den besagten Patienten umfasst.

6. System (1; 101) nach Patentanspruch 5, **dadurch gekennzeichnet, dass** die besagte Schnittstelle (114) eine Anzeige-Einheit (116) für die besagte Patientin/den besagten Patienten umfasst.

7. System (201; 301; 401) nach jeglichem der vorhergehenden Patentansprüche, **dadurch gekennzeichnet, dass** die besagte Managementeinheit (30) und die besagte drahtlose Kommunikationseinheit (40) zu einem zweiten Gerät (230) gehören, vorzugsweise einem Smartphone, besonders bevorzugt einem Smartphone mit einer speziellen APP.

8. System (201; 301; 401) nach Patentanspruch 7, **dadurch gekennzeichnet, dass** die besagte zweite Vorrichtung (230) drahtlos mit der besagten Identifizierungseinheit (20) kommuniziert, die den Zustand der besagten Blisterpackung (10; 410) identifiziert, vorzugsweise durch einen Kommunikationstyp, der zu der Gruppe von Kommunikationstypen gehört, die folgende Funktionen umfassen: BT-Kommunikation, NFC-Kommunikation, ZIGBEE-Kommunikation, WIFI-Kommunikation, UHF-Kommunikation.

9. System (401) nach jeglichem der vorhergehenden Patentansprüche, **dadurch gekennzeichnet, dass** die besagte Identifikationseinheit (20) in die besagte Blisterpackung (10; 410) integriert ist.

10. System (1; 101; 201; 301; 401) nach jeglichem der vorhergehenden Patentansprüche, **dadurch gekennzeichnet, dass** der besagte Dienstanbieter (50) eine oder mehrere Schnittstellen für Drittbetreiber (Ul, U2, U3) verwaltet.

11. System (1; 101; 201; 301; 401) nach jeglichem der vorhergehenden Patentansprüche, **dadurch gekennzeichnet, dass** die besagte drahtlose Kommunikationseinheit (40) und der besagte Dienstanbieter (50) über ein flächendeckendes Kommunikationsnetzwerk miteinander kommunizieren, vorzugsweise eines der Netze GSM, 30, 40, 50, Sigfox, LoRa.

12. System (1; 101; 201; 301; 401) nach jeglichem der vorhergehenden Patentansprüche, **dadurch gekennzeichnet, dass** die besagte Managementeinheit (30) Speichermittel umfasst, die geeignet sind, Daten über die besagte Patientin/den besagten Patienten zu speichern.

13. System (1; 101; 201; 301; 401) nach jeglichem der vorhergehenden Patentansprüche, **dadurch gekennzeichnet, dass** die besagte Blisterpackung (10; 410) ein Identifikationssystem umfasst, das geeignet ist, einen Identifikationscode für die Art des darin aufgenommenen Medikaments zu erzeugen.

14. System (1; 101; 201; 301; 401) nach jeglichem der vorhergehenden Patentansprüche, **dadurch gekennzeichnet, dass** das besagte Gehäuse (14) ein Identifikationssystem umfasst, das geeignet ist, einen Identifikationscode für die Art der Blisterpackung (10; 410) oder des Medikaments, das darin aufgenommenen werden kann, zu erzeugen.

15. System (1; 101; 201; 301; 401) nach Patentanspruch 13 oder 14, **dadurch gekennzeichnet, dass** das besagte Identifikationssystem einen Barcode oder einen RFID-Code oder einen NFC-Code umfasst.

16. System (1; 101; 201; 301; 401) nach einem der Patentansprüche 13 bis 15, **dadurch gekennzeichnet, dass** die besagte Identifikationseinheit (20) Lesemittel umfasst, die geeignet sind, den besagten Identifikationscode zu lesen.

17. System (1; 101; 201; 301; 401) nach Patentanspruch 15, **dadurch gekennzeichnet, dass** die besagten Lesemittel einen Barcodeleser, einen RFID-Leser, einen NFC-Leser umfassen.

18. System (1; 101; 201; 301; 401) nach jeglichem der vorhergehenden Patentansprüche, **dadurch gekennzeichnet, dass** die besagte Managementeinheit (30) und/oder der besagte Dienstanbieter (50) geeignet sind/ist, zu bestimmen, wann die besagten Medikamente (P) aus der besagten Blisterpackung (10; 410) entnommen worden sind.

19. Ein Gehäuse, das geeignet ist, eine Blisterpackung (10) für ein oder mehrere Medikamente aufzunehmen, wobei das besagte Gehäuse (14) Erfassungsmittel (15) umfasst, die geeignet sind, zu erfassen, wann eines der besagten einen oder mehreren Medikamente (P) aus der besagten Blisterpackung (10) entnommen wird/werden,
**wobei** das besagte Gehäuse (14) eine Identifikationseinheit (20) umfasst, die den Zustand der besagten Blisterpackung (10) identifiziert, wobei die besagte Identifikationseinheit (20) geeignet ist, zu identifizieren, wie viele und/oder welche der besagten einen oder mehreren Medikamente (P) aus der besagten Blisterpackung (10) entnommen werden, und
**wobei** das besagte Gehäuse (14) drahtlos mit einer externen Einheit (230) kommuniziert,
**gekennzeichnet dadurch, dass**
das besagte Gehäuse (14) ferner ein erstes Substrat (14a) umfasst, auf das ein Layout (16) mit einer leitfähigen Tinte gedruckt ist und das in einem Format gestanzt ist, das zur Aufnahme der besagten Blisterpackung (10) geeignet ist, das besagte Gehäuse (14) ferner ein zweites Substrat (14b) umfasst, auf dem Löcher (17) definiert sind, wobei die besagten Löcher in einer Position ausgeführt sind, die mit einer Position des einen oder der mehreren Medikamente (P) in der Blisterpackung (10) ausgerichtet ist, wenn die Blisterpackung (10) in dem Gehäuse (14) aufgenommen wird.

## Revendications

1. Système (1 ; 101 ; 201 ; 301 ; 401) pour la surveillance et/ou le contrôle de la prise d'un ou plusieurs médicaments (P) par un patient, lesdits un ou plusieurs médicaments (P) étant contenus dans un emballage blister (10 ; 410), ledit système (1 ; 101 ; 201 ; 301 ; 401) comprenant :
- une unité d'identification (20) indiquée pour identifier la condition dudit emballage blister (10 ; 410), ladite unité d'identification (20) étant indiquée pour identifier combien et/ou quels desdits un ou plusieurs médicaments (P) sont prélevés dudit emballage blister (10 ; 410) ;
- une unité de gestion (30) communiquant avec ladite unité d'identification (20) et indiquée pour recevoir et traiter des informations transmises par ladite unité d'identification (20) et concernant combien et/ou quels médicaments (P) ont été prélevés dudit emballage blister (10 ; 410) ;
- une unité de communication sans fil (40) communiquant avec ladite unité de gestion (30) et indiquée pour transmettre et/ou recevoir des données sans fil ;
- un fournisseur de services (50) indiqué pour recevoir des données sans fil de ladite unité de communication sans fil (40) et/ou pour transmettre des données sans fil à ladite unité de communication sans fil (40) ;
un boîtier (14) indiqué pour recevoir ledit emballage blister (10), ledit boîtier (14) comprenant des moyens de détection (15) indiqués pour détecter combien et/ou quels desdits un ou plusieurs médicaments (P) sont prélevés dudit emballage blister (10),
**caractérisé par**
ledit boîtier (14) comprenant en outre un premier substrat (14a) sur lequel une configuration (16) est imprimée avec une encre de type conductrice et qui est découpée dans un format indiqué pour recevoir ledit emballage blister (10), ledit boîtier (14) comprenant également un deuxième substrat (14b) sur lequel des trous (17) sont définis, lesdits trous étant réalisés dans une position qui est alignée avec la position de l'un ou plusieurs médicaments (P) dans l'emballage blister (10) quand l'emballage blister (10) est reçu dans le boîtier (14).

2. Système (1 ; 101 ; 201) selon la revendication 1, **caractérisé en ce qu'**il comprend un module de connexion (112 ; 212) interposé entre ledit boîtier (14) et ladite unité d'identification (20).

3. Système (301) selon la revendication 1 ou 2, **caractérisé en ce que** ladite unité d'identification (20) fait partie dudit boîtier et communique sans fil avec ladite unité de gestion (30).

4. Système (1 ; 101) selon l'une quelconque des revendications de 1 à 2, **caractérisé en ce que** ledit module de connexion (112), ladite unité d'identification (20), ladite unité de gestion (30) et ladite unité de communication sans fil (40) appartiennent à un premier dispositif (110) qui est indiqué pour être utilisé par ledit patient et pour être reliée audit boîtier (14) recevant ledit emballage blister (10).

5. Système (1 ; 101) selon la revendication 4, **caractérisé en ce que** ledit premier dispositif (110) comprend une interface (114) pour ledit patient.

6. Système (1 ; 101) selon la revendication 5, **caractérisé en ce que** ladite interface (114) comprend une unité d'affichage (116) pour ledit patient.

7. Système (201 ; 301 ; 401) selon l'une quelconque des revendications précédentes, **caractérisé en ce que** ladite unité de gestion (30) et ladite unité de communication sans fil (40) appartiennent à un deuxième dispositif (230), préférablement un smartphone, plus préférablement un smartphone avec une APPLI dédiée.

8. Système (201 ; 301 ; 401) selon la revendication 7, **caractérisé en ce que** ledit deuxième dispositif (230) communique sans fil avec ladite unité d'identification (20) qui identifie la condition dudit emballage blister (10 ; 410), préférablement au moyen d'un type de communication appartenant au groupe de types de communication incluant les communications suivantes : communication BT, communication NFC, communication ZIGBEE, communication WIFI, communication UHF.

9. Système (401) selon l'une quelconque des revendications précédentes, **caractérisé en ce que** ladite unité d'identification (20) est intégrée dans ledit emballage blister (10 ; 410).

10. Système (1 ; 101 ; 201 ; 301 ; 401) selon l'une quelconque des revendications précédentes, **caractérisé en ce que** ledit fournisseur de services (50) gère une ou plusieurs interfaces pour des opérateurs tiers (Ul, U2, U3).

11. Système (1 ; 101 ; 201 ; 301 ; 401) selon l'une quelconque des revendications précédentes, **caractérisé en ce que** ladite unité de communication sans fil (40) et ledit fournisseur de services (50) communiquent entre eux à travers un réseau de communications de large variété, préférablement l'un parmi les réseaux GSM, 30, 40, 50, Sigfox, LoRa.

12. Système (1 ; 101 ; 201 ; 301 ; 401) selon l'une quelconque des revendications précédentes, **caractérisé en ce que** ladite unité de gestion (30) comprend des moyens de stockage indiqués pour stocker des données concernant ledit patient.

13. Système (1 ; 101 ; 201 ; 301 ; 401) selon l'une quelconque des revendications précédentes, **caractérisé en ce que** ledit emballage blister (10 ; 410) comprend un système d'identification indiqué pour générer un code d'identification pour le type de médicament reçu dans l'emballage même.

14. Système (1 ; 101 ; 201 ; 301 ; 401) selon l'une quelconque des revendications précédentes, **caractérisé en ce que** ledit boîtier (14) comprend un système d'identification indiqué pour générer un code d'identification afin d'identifier le type d'emballage blister (10 ; 410) ou le type de médicament qu'il peut recevoir.

15. Système (1 ; 101 ; 201 ; 301 ; 401) selon la revendication 13 ou 14, **caractérisé en ce que** ledit système d'identification comprend un code-barres ou un code RFID ou un code NFC.

16. Système (1 ; 101 ; 201 ; 301 ; 401) selon l'une quelconque des revendications de 13 à 15, **caractérisé en ce que** ladite unité d'identification (20) comprend des moyens de lecture indiqués pour lire ledit code d'identification.

17. Système (1 ; 101 ; 201 ; 301 ; 401) selon la revendication 15, **caractérisé en ce que** lesdites moyens de lecture comprennent un lecteur de code-barres, un lecteur RFID, un lecteur NFC.

18. Système (1 ; 101 ; 201 ; 301 ; 401) selon l'une quelconque des revendications précédentes, **caractérisé en ce que** ladite unité de gestion (30) et/ou ledit fournisseur de services (50) est/sont indiqués pour déterminer quand lesdits médicaments (P) ont été prélevés dudit emballage blister (10 ; 410).

19. Boîtier indiqué pour recevoir un emballage blister (10) pour un ou plusieurs médicaments (P), ledit boîtier (14) comprenant des moyens de détection (15) indiqués pour détecter quand un ou plusieurs desdits médicaments (P) est prélevé dudit emballage blister (10),
où ledit boîtier (14) comprend une unité d'identification (20) qui identifie la condition dudit emballage blister (10), ladite unité d'identification (20) étant indiquée pour identifier combien et/ou quels desdits un ou plusieurs médicaments (P) sont prélevés dudit emballage blister (10) et
où ledit boîtier (14) communique sans fil avec une unité extérieure (230) **caractérisée par**
ledit boîtier (14) comprenant en outre un premier substrat (14a) sur lequel une configuration est imprimée avec une encre de type conductrice et qui est découpée dans un format indiqué pour recevoir ledit emballage blister (10), ledit boîtier (14) comprenant en outre un deuxième substrat (14b) sur lequel des trous sont définis, lesdits trous étant réalisés dans une position qui est alignée avec une position de l'un ou plusieurs médicaments (P) dans l'emballage blister (10) quand l'emballage blister (10) est reçu dans le boîtier (14).
